(19) Europäisches Patentamt
European Patent Office
Office européen des brevets

(11) Publication number: 0 304 244
A2

(12) # EUROPEAN PATENT APPLICATION

(21) Application number: 88307492.4

(22) Date of filing: 12.08.88

(51) Int. Cl.[4]: C 07 C 93/04
C 07 D 295/08,
C 07 D 213/02,
C 07 D 277/22,
C 07 D 213/80, A 61 K 31/13,
A 61 K 31/44, A 61 K 31/425,
A 61 K 31/445

(30) Priority: 18.08.87 JP 204795/87

(43) Date of publication of application:
22.02.89 Bulletin 89/08

(84) Designated Contracting States:
AT BE CH DE ES FR GB GR IT LI LU NL SE

(71) Applicant: Takeda Chemical Industries, Ltd.
27, Doshomachi 2-chome Higashi-ku
Osaka-shi Osaka, 541 (JP)

(72) Inventor: Inoue, Keizo
17-605, 3 Etchujima 1-chome
Koto-ku Tokyo 135 (JP)

Nomura, Hiroaki
9-15, Higashikanmaki 3-chome
Takatsuki Osaka 569 (JP)

Tasaka, Akihiro
20-102, 9 Yamadahigashi 2-chome
Suita Osaka 565 (JP)

(74) Representative: Lewin, John Harvey et al
ELKINGTON AND FIFE Beacon House 113 Kingsway
London WC2B 6PP (GB)

(54) **Propanediol derivatives, their production and use.**

(57) Novel compounds having the formula:
$R^1-OCH_2CH_2CH_2O-(CH_2)_n-R^2$
wherein $R^1$ stands for a higher alkyl group; $R^2$ stands for a primary to tertiary amino group or a quaternary ammonium group; and n denotes an integer of 3 to 10; and salts thereof, have excellent anti-tumor action and therefore are useful as anti-tumor agents.

EP 0 304 244 A2

**Description**

## PROPANEDIOL DERIVATIVES, THEIR PRODUCTION AND USE

Industrial Field of the Utilization

This invention relates to propanediol derivatives useful as medicines. More specifically, the present invention relates to compounds represented by the formula:

$R^1$-$OCH_2CH_2CH_2O$-$(CH_2)_n$-$R^2$ [I]

[wherein $R^1$ stands for a higher alkyl group; $R^2$ stands for a primary to tertiary amino group or a quaternary ammonium group; and n denotes an integer of 3 to 10] and salts thereof.

Prior Art

Recently, physiological activities of lipid derivatives have attracted attention. For example, platelet activating factor shown by the formula [II] [hereinafter abbreviated as PAF] has attracted attention as an important chemical mediator in living bodies, because it shows a variety of strong physiological actions such as platelet-activating action, vessel permeability-enhancing action and blood pressure-lowering action [C. A. Demopoulos, R. N. Pinckard, and D. J. Hanahan, J. Biol. Chem., 254, 9355 (1979); J. Benveniste et al., C. R. Acad. Sci(D), 289, 1037 (1979)].

Besides, the phospholipid shown by the formula [III], which is an analog to the compounds of the formula [II], is known to have an anti-tumor action. [W. E. Berdel et al., Anticancer Res. 1, 345 (1081); the official gazette of Japanese Unexamined Patent Publication No. 134027/1977]. However, this compound has such side effects as platelet aggregation and hypotensive action due to its structural similarity to PAF. [D. J. Hanahan et al., Biochem. Biophys. Res. Commun., 99, 183 (1981)].

On the other hand, lipids having no phosphate group, represented by the formula [IV] [wherein $R^3$ = long chain alkyl; $R^4$ = short chain or long chain alkyl or acyl having 2 to 5 carbon atoms; X = $O(CH_2)_m$-$R^5$; m = an integer of 1 to 12; and $R^5$ = amino group, acylamino group or quaternary ammonium group] were disclosed to exhibit antagonism to PAF, and to be possibly used as a therapeutic agent for disorders due to PAF. [the official gazette of Japanese Unexamined Patent Publication No. 100544/1985; the official gazette of Japanese Unexamined Patent Publication No. 198445/1983; T. Miyamoto et al., Kyoto Conference of Prostaglandins, Nov. 26-28, 1984, Abst. p.99].

However, no concrete disclosure has yet been made in respect of anti-tumor actions.

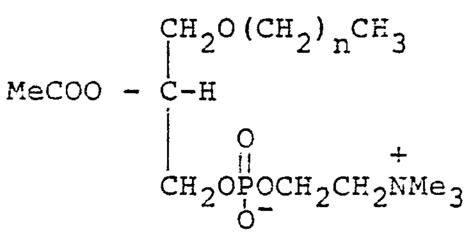

[II]
(n=15, 17)

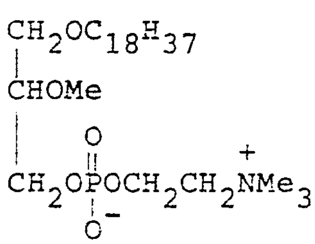

[III]

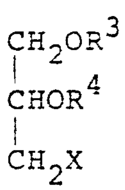

[IV]

## Problems that the Invention is to solve

In recent years, it has been gradually elucidated that the characteristics of tumor cells, such as abnormal proliferation of cells or disorders of intracellular interaction, are derived from structural and functional abnormalities of the carcinoma cell's membrane.

On the other hand, it has become apparent that amphipathic lipids have affinity to cell membrane and possibly exert various influences on cellular metabolism and functions.

The present inventors supposed that, among amphipathic lipids, there is a possibility of existence of compounds having a high selectivity to tumor cells and useful as anti-tumor agents, and they have diligently conducted intensive study.

There have already been found several reports that tumor cells are inferior to normal cells in the alkylether cleavage activity [Wykle, R.L. and Snyder, F., The Enzymes of Biological Membranes, Martonosi, A., Ed., Vol. 2, Plenum Press, New York, 1976, 87; Lin, H. J., Ho, F. C. S., and Lee, C. L. H., Cancer Res., 38, 946 (1978); Modolell, M., Andreesen, R., Pahlke, W., Brugger, U., and Munder, P.G., Cancer Res., 39, 4681 (1976)], and therefore it is expected that lipids having an alkylether group are accumulated in tumor cells more than normal cells because of slow metabolism in tumor cells, and then the lipids show selective cytotoxicity.

Further, it is known that membrane electric potential of tumor cells is lower than that of normal cells due to increase of sialate on the surface of membrane, and it is expected that cationic lipids show higher affinity with tumor cells. [T. J. Lampidis et al., Biomedicine & Pharmacotherapy, 39, 220(1985)]. The present inventors have found that the cationic lipids having an ether-linkage represented by the general formula [I] exhibit a strong inhibitory effect on proliferation of tumor cells, the mechanism of which is different from that of known carcinostatic agents attacking DNA directly, with less undesirable side effects, thus the present invention being completed.

## Means of Solving the Problems

The present inventors have diligently conducted intensive studies aiming at development of a drug having an excellent antitumor action but showing no PAF-like action, and have found that the compounds of the general formula [I] show a strong inhibitory effect on proliferation of tumor cells but have neither platelet-aggregating nor blood pressure-lowering actions, the side effects being thus diminished, and the present invention has been completed.

More specifically, the present invention is to provide compounds represented by the general formula:

$R^1$-$OCH_2CH_2CH_2O$-$(CH_2)_n$-$R^2$ [I]

[wherein $R^1$ stands for a higher alkyl group; $R^2$ stands for a primary to tertiary amino group or a quaternary ammonium group; and n denotes an integer of 3 to 10] and salts thereof.

In the above-mentioned general formula [I], the higher alkyl group represented by $R^1$ includes straight-chain alkyl groups having 8 to 22 carbon atoms, such as n-octyl, n-nonyl, n-decyl, n-undecyl, n-dodecyl, n-tetradecyl, n-hexadecyl, n-heptadecyl, n-octadecyl and n-eicosyl, and branched chain alkyl groups having 8 to 22 carbon atoms, such as 18,18-dimethylnonadecyl, 19,19-dimethyleicosyl, 19-methyleicosyl, 3,7,11-trimethyldodecyl and 3,7,11,15-tetramethylhexadecyl. The said higher alkyl groups may have a substituent such as cycloalkyl, aryl, halogen (about 1 to 7), cyano, ethynyl, oxo or lower alkoxy. The said lower alkoxy group includes alkoxy groups of 1 to 4 carbon atoms, such as methoxy and ethoxy. The said cycloalkyl group includes 3- to 8-membered cycloalkyl groups such as cyclopentyl and cyclohexyl. The said aryl group includes phenyl, and the said phenyl group may be substituted with lower($C_{1-4}$)alkyl, lower($C_{1-4}$)alkoxy, hydroxy, nitro or halogen. Halogens as substituents of the above-mentioned higher alkyl group and the phenyl group include fluorine, bromine and chlorine. When $R^1$ is a substituted higher alkyl group, the position at which the substitution occurs may be any substitutive position of the higher alkyl group. When the substituent is cycloalkyl, aryl, halogen, cyano or ethynyl, the substitutive position is preferably ω (omega)-position of the higher alkyl group. The said substituted alkyl groups include, among others, 13-cyclopentyltridecyl, 12-cyclohexyldodecyl, 10-cyclohexyldecyl, 12-phenyldodecyl, octadecan-17-ynyl, hexadecan-15-ynyl, 2-oxooctadecyl, 16-cyanohexadecyl, 2-methoxyeicosyl, 2-methoxyoctadecyl, 18-chlorooctadecyl, 18,18-dichlorooctadecyl, 18,18,18-trifluorooctadecyl, 18,18,18-trichlorooctadecyl, 14,14,15,15,16,16,16-heptafluorohexadecyl and 16,16,17,17,18,18,18-heptafluorooctadecyl.

The primary to tertiary amino group represented by $R^2$ includes groups represented by the formula:

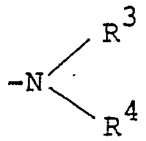

[wherein $R^3$ and $R^4$ independently stand for hydrogen or a lower alkyl group, or $R^3$ and $R^4$, taken together with the adjacent nitrogen atom, form a cyclic amino group].

The lower alkyl group represented by $R^3$ or $R^4$ includes alkyl groups having 1 to 5 carbon atoms, such as

methyl, ethyl, propyl, butyl and pentyl, among which a methyl group is preferable.

The cyclic amino group includes 5- or 6-membered cyclic amino groups, such as pyrrolidino, piperidino, piperazino and morpholino, and these groups may be substituted with a substituent such as a lower alkyl group having 1 to 4 carbon atoms (e.g. methyl, ethyl, propyl, butyl), a hydroxyl group, a hydroxyethyl group, an aminoethyl group, a carboxy group, a lower($C_{1-4}$)alkoxycarbonyl group, a carbamoyl group or a ureido group.

The quaternary ammonium group represented by $R^2$ includes groups shown by the formula:

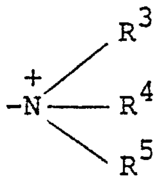

[wherein $R^3$, $R^4$ and $R^5$ independently stand for hydrogen or a lower alkyl group or

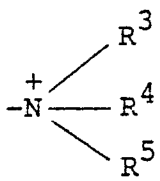

stands for a cyclic ammonium group].

The lower alkyl group represented by $R^3$, $R^4$ or $R^5$ includes alkyl groups having 1 to 5 carbon atoms (e.g. methyl, ethyl, propyl, butyl, pentyl), among which a methyl group is preferable.

The cyclic ammonium group includes pyridinio, oxazolio, thiazolio, pyridazinio, quinolinio, isoquinolinio, 1-[lower($C_{1-4}$)alkyl]pyrrolidinio, 1-[lower($C_{1-4}$)alkyl]piperidinio, N-[lower($C_{1-4}$)alkyl]morpholinio and 1-[lower($C_{1-4}$)alkyl]piperazinio, and these groups may be further substituted with a substituent such as a lower alkyl group having 1 to 4 carbon atoms (e.g. methyl, ethyl, propyl, butyl), a hydroxyl group, a hydroxyethyl group, an aminoethyl group, a carboxy group, a lower($C_{1-4}$)alkoxycarbonyl group, a carbamoyl group or a ureido group.

n denotes an integer of 3 to 10, preferably an integer of 3 to 8 and more preferably an integer of 4 to 6.

When $R^2$ is a primary to tertiary amino group, the compound [I] may form a pharmaceutically acceptable salt with an inorganic acid (e.g. hydrochloric acid, hydrobromic acid, hydriodic acid, sulfuric acid) or an organic acid (e.g. acetic acid, lactic acid, tartaric acid, citric acid, methanesulfonic acid, ethanesulfonic acid, benzenesulfonic acid, toluenesulfonic acid).

When $R^2$ is a quaternary ammonium group, the compound [I] forms a salt with an anion ($X^-$) such as a pharmaceutically acceptable anion of an inorganic acid (e.g. hydrochloric acid, hydrobromic acid, hydriodic acid, sulfuric acid) or an organic acid (e.g. acetic acid, lactic acid, tartaric acid, citric acid, methanesulfonic acid, ethanesulfonic acid, benzenesulfonic acid, toluenesulfonic acid) or forms an intramolecular salt with an anion in the molecule. Desirable anions include a halogen ion (e.g. chlorine ion, bromine ion, iodine ion), an anion of methanesulfonic acid, and an anion of p-toluenesulfonic acid. The anion in the molecule includes a carboxylato group (carboxy group) and an oxido group (hydroxy group).

The compound [I] of the present invention can be produced by, for example, the following methods.

$$R^1\text{-}OCH_2CH_2CH_2O\text{-}(CH_2)_n\text{-}Y \qquad [IX]$$

amine

[I]

[wherein Y stands for benzenesulfonyloxy, lower($C_{1-4}$)alkylbenzenesulfonyloxy, lower($C_{1-4}$)alkanesulfonyloxy or halogen (e.g. bromine, chlorine), and the other symbols are of the same meaning as defined above].

The compound [I] can be obtained by allowing the compound [IX] to react with an amine

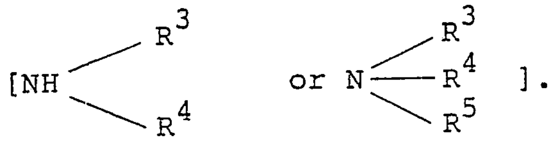

The said reaction is conducted, using an excess amount of the amine, in said amine or a suitable solvent (e.g. water, ethanol, toluene, tetrahydrofuran, etc. or a mixture thereof) at -20°C to +150°C, preferably at 0°C to +80°C. Separation and purification of the compound [I] from the reaction mixture can be performed by known procedures such as solvent distillation, solvent-extraction and column chromatography (silica gel).

The starting compound [IX] can be produced by, for example, the procedure shown below.

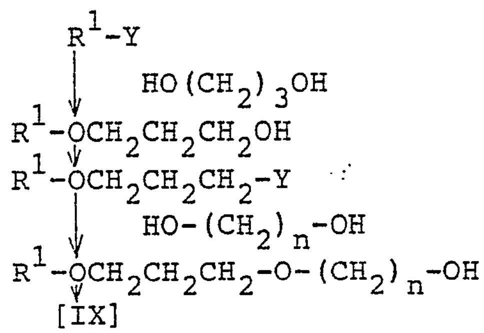


[V]

[VI]

[VII]

[VIII]

The compound [IX] can be obtained by allowing the compound [VIII] to react with a sulfonyl halide in a suitable anhydrous solvent (e.g. benzene, toluene, dichloromethane, chloroform, pyridine, etc., or a mixture thereof) in the presence of a base (e.g. tertiary amines such as triethylamine and pyridine) at -20°C to +100°C, preferably at -10°C to +50°C [Y = benzenesulfonyloxy, lower alkylbenzenesulfonyloxy or lower alkanesulfonyloxy]. The sulfonyl halide to be employed include, for example, benzenesulfonyl halide (e.g. benzenesulfonyl chloride), a lower($C_{1-4}$)alkylbenzenesulfonyl halide (e.g. toluenesulfonyl chloride) and a lower($C_{1-4}$)alkanesulfonyl halide (e.g. methanesulfonyl chloride). The compound [IX] (Y = halogen) can be obtained by allowing the compound [VIII] to react with an anhydrous hydrogen halide (e.g. hydrogen chloride, hydrogen bromide), thionyl halide (e.g. thionyl chloride, thionyl bromide), phosphorus halide (e.g. phosphorus pentachloride, phosphorus trichloride, phosphorus pentabromide, phosphorus tribromide) or phosphorus oxychloride in an inert solvent (e.g. dichloromethane) at -20°C to +50°C.

The compound [IX], wherein Y stands for halogen, can also be obtained by allowing an alkali metal halide (e.g. sodium bromide, sodium iodide) to react with the compound [IX] wherein Y stands for benzenesulfonyloxy, lower alkylbenzenesulfonyloxy or lower alkanesulfonyloxy.

The compound [VIII] can be obtained by allowing the compound [VII] to react with the compound represented by the formula:

$HO(CH_2)_n OH$ .

The reaction is conducted in a suitable solvent (e.g. benzene, toluene, hexane, dioxane, tetrahydrofuran, etc.) in the presence of a strong base (e.g. sodium hydroxide, potassium hydroxide, etc., or an aqueous solution thereof), and, when the conditions are hydrous, preferably in the presence of a phase-transfer catalyst (e.g. cetyltrimethylammonium chloride, benzyltrimethylammonium chloride), at -20°C to +150°C, preferably +20°C to +100°C.

The reaction of [VI] → [VII] can be conducted in the same manner as that of [VIII] → [IX], and the reaction of [V] → [VI] can be conducted in the same manner as that of [VII] → [VIII].

The compound [VIII] can also be produced by the following method.

$$Y-(CH_2)_n-OR \qquad [X]$$

$$\downarrow \quad HO-(CH_2)_3-OH$$

$$HO-CH_2CH_2-CH_2-O-(CH_2)_n-OR \qquad [XI]$$

$$\downarrow \quad R^1-Y$$

$$R^1-OCH_2CH_2CH_2-O-(CH_2)_n-OR \qquad [XII]$$

[VIII]

[wherein R stands for a hydroxyl-protecting group such as a trityl group, a benzyl group or a tetrahydropyranyl group, and the other symbols are of the same meaning as defined above].

The reactions of [X] → [XI] and [XI] → [XII] can be conducted in the same manner as that of the reaction of [VII] → [VIII].

The reaction of [XII] → [VIII] is a reaction to remove the protecting group. When R is a trityl group or a tetrahydropyranyl group, the compound [XII] is subjected to a reaction in the presence of an acid (e.g. hydrochloric acid, acetic acid, trifluoroacetic acid, p-toluenesulfonic acid, etc.), in a suitable aqueous solvent (e.g. methanol, ethanol, dioxane, etc. or a mixture thereof), at -10°C to +120°C, preferably at 0°C to +80°C.

When R is a benzyl group, the compound [VIII] can be obtained by subjecting the compound [XII] to hydrogenolysis in a suitable solvent (e.g. alcohol, aqueous acetic acid, etc.) in the presence of a suitable catalyst (e.g. palladium-carbon).

The compound [XII] can also be obtained by allowing the compound [VI] to react with the compound [X] in the same manner as the reaction of [VII] → [VIII].

The compound [IX] wherein Y is halogen can also be produced by, for example, the following process.

[VI]

$$\downarrow \quad Y-(CH_2)_n-Y$$

[IX]

[wherein Y stands for halogen]

This reaction can be conducted in the same manner as that of [VII] → [VIII].

Compounds obtained by the respective processes mentioned above can, upon necessity, be separated and purified by means of silica gel column chromatography. Eluents include a hexane-ethyl acetate mixture, a dichloromethane-ethyl acetate mixture, etc.

When $R^2$ is a primary to tertiary amino group, the salt of the compound [I] may, in some instances, be obtained by the above-mentioned production methods, but, when necessary, it can be obtained by addition of an inorganic or organic acid.

When $R^2$ is a quaternary ammonium group, the anion($X^-$) can be exchanged with another anion by using anion-exchange resin, if necessary.

Representative methods of production of the compound (I) are described above, but the methods of production of the compound (I) should not be limited only to these.

## Effect

Antitumor activity of the compounds of the present invention is shown by Test Examples given later.

The compounds [I] have a remarkable cell-killing action against tumor cells and exhibit remarkable antitumor effects by administration to cancer-bearing warm-blooded animals. Further, the compounds [I] have no platelet activating action (PAF action), thus exhibiting no side-effect caused by PAF action (e.g. platelet aggregation, blood pressure lowering action, etc.).

Schedule, route and dosage of the administration can be selected according to the subject and symptoms to be treated; the dose for a mammal is usually about 0.1 to 150 mg/kg (body weight)/day, preferably 2 to 40 mg/kg (body weight)/day, on the compound (I) basis. Frequency of administration of the compound is about 1 to 3 times a day, or at the intervals of 2 to 7 days. The compound can also be administered by intravenous drip infusion over a long period to maintain the level of the compound over a long period. In parenteral administration of the compound, further improvement in safety, for example, prevention of tissue (local) impairment without affecting the efficacy, can be expected by combination with serum albumin or various globulins.

The compounds [I] and the salts thereof are excellent both in hydrophilic and in lipophilic properties, with

low toxicities, and therefore can be safely administered orally or parenterally to mammals as powders as they are or as a pharmaceutical composition in a suitable dosage form.

Pharmaceutical compositions used for the administration contain an effective amount of the compound [I] or a salt thereof and a pharmaceutically acceptable carrier or excipient.

Injections for the parenteral administration by this invention include sterilized aqueous or non-aqueous solutions, suspensions and emulsions. Aqueous solutions and suspensions include distilled water for injection and physiological saline. Non-aqueous solutions and suspensions include Intralipids, propylene glycol, vegetable oils such as olive oil, alcohols such as ethanol, and polysorbate 80, etc. Such compositions may contain additionally adjuvants such as antiseptics, moistening agents, emulsifiers and dispersants, and aqueous injections may contain adjuvants such as glucose, serum albumin, serum (plasma) globulins, etc. These preparations are sterilized by, for example, filtration through a bacteria-retention filter, combination of a disinfectant or ultraviolet ray irradiation. Sterilized solid preparations are also produced, which are dissolved in sterilized water or sterilized solvent for injection prior to use. Tablets and capsules can be prepared in accordance with conventional methods. To prepare these solid compositions, the compound [I] or a salt thereof is mixed with at least one inactive carrier or excipient such as lactose, mannitol, glucose, hydroxypropyl cellulose, microcrystalline cellulose or starch. The compositions may contain an additive other than inactive carriers or excipients, for example, a lubricant such as magnesium stearate or a disintegrator such as calcium cellulose gluconate.

## Examples

In the following, the present invention is illustrated in more detail by Working Examples and Reference Examples, but this invention should not be limited to these Examples.

### Reference Example 1

Synthesis of 3-(octadecyloxy)propyl methanesulfonate:

In dichloromethane (50 ml) is dissolved 3-(octadecyloxy)propanol (8.0 g). To the solution are added, under ice-cooling, methanesulfonyl chloride (4.18 g) and triethylamine (5.1 ml). The mixture is stirred at room temperature for one hour. The reaction mixture is washed with water and an aqueous solution of sodium hydrogencarbonate, successively, and then dried. The solvent is distilled off under reduced pressure to give the above-titled compound (9.7 g).
IR(KBr)$cm^{-1}$: 2925, 2852, 1468, 1352, 1330, 1162, 1120, 970, 952, 948
NMR(90MHz, $CDCl_3$)δ: 0.87 (3H, t), 1.25 (32H, m), 1.95 (2H, m), 2.97 (3H, s), 3.3 to 3.85 (4H, m), 4.33 (2H, t)

### Reference Example 2

Synthesis of 4-[3-(octadecyloxy)propoxy]butanol:

Sodium hydride (1.2 g) is heated at 70°C for 10 minutes in dimethyl sulfoxide (50 ml), to which is added 1,4-butanediol (4.5 g). The mixture is heated for further one hour. To this reaction mixture is added dropwise a solution of 3-(octadecyloxy)propyl methanesulfonate obtained in Reference Example 1 (4.06 g) in dimethyl sulfoxide (15 ml), and the mixture is stirred at 70°C for one hour. The reaction mixture is poured into cold water, and the mixture is subjected to extraction with a mixture of hexane and ethyl acetate. The extract is washed with water and then dried, and the solvents are then distilled off under reduced pressure. The residue is subjected to purification by means of silica gel column chromatography to obtain the above-titled compound (2.2 g).
IR(KBr)$cm^{-1}$: 3425, 3350, 2925, 2852, 1492, 1470, 1372, 1350, 1120, 960
NMR(90MHz, $CDCl_3$) δ: 0.87 (3H, t), 1.1 to 2.1 (38H, m), 3.1 to 3.6 (10H, m)

### Reference Example 3

Synthesis of 4-[3-(octadecyloxy)propoxy]butyl methanesulfonate:

Using the alcohol compound obtained in Reference Example 2 (2.2 g), mesylation is conducted in the same manner as that in Reference Example 1 to obtain the above-titled compound (2.6 g).
IR(KBr)$cm^{-1}$: 2925, 2850, 1468, 1350, 1330, 1160, 1120, 970, 950
NMR(90MHz, $CDCl_3$) δ: 0.87 (3H, t), 1.1 to 2.1 (38H, m), 2.97 (3H, s), 3.1 to 3.8 (3H, m), 4.3 (2H, t)

Working Example 1

Synthesis of N-[4-[3-(octadecyloxy)propoxy]butyl]N,N,N-trimethylammonium methanesulfonate:

A solution of the mesylate obtained in Reference Example 3 (1.3 g) in toluene (10 ml) containing trimethylamine (2 g) is stirred for 3 days at room temperature, and further stirred for 8 hours at 40°C. The reaction mixture is concentrated, and the residue is subjected to purification by means of silica gel chromatography (eluent: chloroform-methanol-water = 65:25:4). Reprecipitation from chloroform-acetone gives the above-titled compound as colorless powder (1.0 g).
IR(KBr)$cm^{-1}$: 2925, 2855, 1470, 1210, 1190, 1115, 1060
NMR(90MHz, $CDCl_3$) δ: 0.87 (3H, t), 1.25 (32H, m), 1.4 to 2.0 (6H, m), 2.73 (3H, s), 3.31 (9H, s), 3.25 to 3.65 (10H, m)

Working Example 2

Synthesis of N-[4-[3-(octadecyloxy)propoxy]butyl]pyridinium methanesulfonate:

A solution of the mesylate obtained in Reference Example 3 (1.3 g) in pyridine (6 ml) is heated at 80°C for 16 hours. The reaction mixture is concentrated, and the residue is subjected to reprecipitation from chloroform-acetone to obtain the above-titled compound (1.36 g).
IR(KRr)$cm^{-1}$: 2915, 2850, 1630, 1488, 1465, 1370, 1205, 1190, 1110, 1050, 785
NMR(90 MHz, $CDCl_3$) δ: 0.87 (3H, t), 1.25 (32H, m), 1.4 to 2.25 (6H, m), 2.75 (3H, s), 3.3 to 3.55 (8H, m), 4.90 (2H, t), 8.07 (2H), 8.45 (1H), 9.26 (2H)

Reference Example 4

Synthesis of 1-bromo-6-[3-(octadecyloxy)propoxy]hexane:

3-(Octadecyloxy)propanol (5.0 g), 1,6-dibromohexane (11.1 g) and cetyltrimethylammonium chloride (0.97 g) are added to toluene (60 ml). To the mixture is added a 50% aqueous solution of sodium hydroxide (12.2 g), and the resulting mixture is stirred at 80°C for 7 hours. After the reaction, the organic layer is separated, and the aqueous layer is subjected to extraction with n-hexane. The organic layers are combined, washed with dilute hydrochloric acid, dried and concentrated. The residue is subjected to purification by means of silica gel chromatography to obtain the above-titled compound as colorless solid (2.44 g).
IR(film)$cm^{-1}$: 2940, 2870, 1470, 1370, 1120, 720
NMR(90MHz, $CDCl_3$) δ: 0.86 (3H), 1.02 to 2.19 (42H), 3.12 to 3.62 (10H)

Reference Example 5

Synthesis of 1-bromo-3-[3-(octadecyloxy)propoxy]propane:

Employing 3-(octadecyloxy)propanol (5 g), 1,3-dibromopropane (9.2 g) and cetyltrimethylammonium chloride (0.97 g), a reaction and aftertreatment are conducted in the same manner as those in Reference Example 4 to obtain the above-titled compound (0.53 g).
IR(film)$cm^{-1}$: 2940, 2870, 1470, 1370, 1120
NMR(90MHz, $CDCl_3$) δ: 0.88 (3H), 1.03 to 2.27 (36H), 3.23 to 3.67 (10H)

Working Example 3

Synthesis of 6-[3-(octadecyloxy)propoxy]hexyl-N,N,N-trimethylammonium bromide:

The bromide obtained in Reference Example 4 (1.45 g) is stirred in toluene (10 ml) containing trimethylamine (2 g) at 50°C for 63 hours. The reaction mixture is concentrated under reduced pressure, and the residue is subjected to reprecipitation from a mixture of chloroform-acetone-ether to give the above-titled compound as colorless powder (1.43 g).
IR($CHCl_3$)$cm^{-1}$: 2920, 2850, 1460, 1360, 1220, 1100
NMR(90MHz, $CDCl_3$) δ: 0.87 (3H), 1.07 to 1.97 (42H), 3.27 to 3.76 (10H), 3.45 (9H)

Working Example 4

Synthesis of 3-[3-(octadecyloxy)propoxy]propyl-N,N,N-trimethylammonium bromide:

Employing the bromide obtained in Reference Example 5 (0.44 g), a reaction is conducted in the same manner as that in Working Example 4 to give the above-titled compound (0.35 g).
IR $(CHCl_3)m^{-1}$: 2920, 2850, 1460, 1370, 1220, 1100
NMR(90MHz, $CDCl_3$) δ: 0.85 (3H), 1.12 to 2.25 (36H), 3.22 to 3.82 (10H), 3.48 (9H)

Reference Example 6

Synthesis of 1-bromo-8-[3-(octadecyloxy)propoxy]octane:

Using 3-(octadecyloxy)propanol (5 g), 1,8-dibromooctane (12.7 g), cetyltrimethylammonium chloride (0.96 g) and a 50% aqueous solution of sodium hydroxide (12 g), a reaction is conducted in the same manner as that in Reference Example 4 to give the above-titled compound as colorless solid (4.2 g).
IR(KBr)$cm^{-1}$: 2925, 2850, 1490, 1462, 1380, 1110
NMR(90MHz, $CDCl_3$) δ: 0.87 (3H, t), 1.10 to 2.0 (46H, m), 3.36 to 3.73 (10H, m)

Reference Example 7

Synthesis of 1-bromo-5-[3-(octadecyloxy)propoxy]pentane:

Employing 3-(octadecyloxy)propanol (5 g), 1,5-dibromopentane (10.8 g), cetyltrimethylammonium chloride (0.96 g) and a 50% aqueous solution of sodium hydroxide (12 g), a reaction and aftertreatment are conducted in the same manner as those in Reference Example 4 to obtain the above-titled compound as colorless wax (3.75 g).
IR(KBr)$cm^{-1}$: 2930, 2850, 1490, 1468, 1370, 1115
NMR(90MHz, $CDCl_3$) δ: 0.87 (3H, t), 1.0 to 2.0 (40H, m), 3.25 to 3.56 (10H, m)

Working Example 5

Synthesis of N-[8-[3-(octadecyloxy)propoxy]octyl]N,N,N-trimethylammonium chloride:

In dioxane (15 ml) is dissolved 1-bromo-8-[3-(octadecyloxy)propoxy]octane obtained in Reference Example 6 (1.2 g). To the solution is added a 30% aqueous solution of trimethylamine, and the reaction mixture is heated at 60°C for 20 hours. The reaction mixture is concentrated and the residue is dissolved in dichloromethane. The solution is washed with an aqueous solution saturated with sodium chloride, dried and concentrated. The residue is subjected to reprecipitation from chloroform-acetone to give the above-titled compound as colorless powder (1.06 g).
IR(KBr)$cm^{-1}$: 3430, 2925, 2852, 1492, 1485, 1380, 1112
NMR(90MHz, $CDCl_3$) δ: 0.87 (3H, t), 1.0 to 1.90 (54H, m), 3.45 (9H, s), 3.26 to 3.70 (10H, m)

Working Example 6

Synthesis of N-[8-[3-(octadecyloxy)propoxy]octyl]thiazolium chloride:

1-Bromo-8-[3-(octadecyloxy)propoxy]octane obtained in Reference Example 6 (0.3 g) is heated in thiazole (0.5 ml) at 80°C for 13 hours. The reaction mixture is concentrated and the residue is dissolved in dichloromethane. The solution is washed with an aqueous solution saturated with sodium chloride, dried and concentrated. The residue is subjected to reprecipitation from chloroform-acetone to give the above-titled compound as pale brown powder (0.23 g).
IR(KBr)$cm^{-1}$: 2925, 2852, 1645, 1550, 1495, 1468, 1380, 1118
NMR(90MHz, $CDCl_3$) δ: 0.87 (3H, t), 1.1 to 2.1 (46H, m), 3.25 to 3.60 (8H, m), 4.84 (2H, t), 8.33 (2H, m), 11.56 (1H)

Working Example 7

Synthesis of N-[5-[3-(octadecyloxy)propoxy]pentyl]N,N,N-trimethylammonium chloride:

1-Bromo-5-[3-(octadecyloxy]propoxy]pentane obtained in Reference Example 7 (1.5 g) is dissolved in toluene (6 ml) containing trimethylamine (1.2 g). The reaction mixture is stirred at 50° C for 24 hours and then concentrated. The residue is dissolved in dichloromethane, and the solution is washed with an aqueous solution saturated with sodium chloride, dried and concentrated. The residue is subjected to reprecipitation from chloroform-acetone to give the above-titled compound as colorless solid (1.50 g).
IR(KBr)$cm^{-1}$: 3450, 2925, 2852, 1480, 1465, 1380, 1115
NMR(90MHz, $CDCl_3$) δ: 0.87 (3H, t), 1.0 to 2.0 (40H, m), 3.20 to 3.70 (10H, m), 3.48 (9H, s)

Working Example 8

Synthesis of N-[5-[3-(octadecyloxy)propoxy]pentyl]pyridinium chloride:

In pyridine (4 ml) is dissolved 1-bromo-5-[3(octadecyloxy)propoxy]pentane obtained in Reference Example 7 (1.5 g). The reaction mixture is stirred at 90° C for 15 hours, and then concentrated. The residue is dissolved in dichloromethane, and the solution is washed with an aqueous solution saturated with sodium chloride, dried and concentrated. The residue is subjected to reprecipitation from chloroform-acetone to give the above-titled compound as colorless solid (0.90 g).
IR(KBr)$cm^{-1}$: 3425, 2925, 2852, 1632, 1490, 1464, 1380, 1115
NMR(90MHz, $CDCl_3$) δ: 0.87 (3H, t), 1.10 to 2.4 (40H, m), 3.30 to 3.56 (8H, m), 5.21 (2H, t), 8.33 (2H), 8.50 (1H), 9.63 (2H, d)

Working Example 9

Synthesis of 1-N-[6-[3-(octadecyloxy)propoxy]hexyl]pyridinio-3-carboxylate:

1-Bromo-6-[3-(octadecyloxy)propoxy]hexane obtained in Reference Example 4 (1.0 g) is heated with ethyl nicotinate (0.66 g) at 90° C for 19 hours. After the reaction mixture is cooled, ether is added to the reaction mixture and the precipitate is collected by filtration to give 1-N-[6-[3-(octadecyloxy)propoxy]hexyl]-3-ethoxycarbonylpyridinium bromide (1.0 g). To this compound in methanol (25 ml) is added with stirring 2N sodium hydroxide (4 ml), and the mixture is stirred at room temperature for 20 minutes. The reaction mixture is neutralized with 1N hydrochloric acid, concentrated under reduced pressure, and extracted with dichloromethane. The extract is washed with an aqueous solution saturated with sodium chloride, dried and concentrated. The residue is subjected to purification by means of silica gel chromatography (chloroform-methanol-water = 65:25:4) and then to reprecipitation from chloroform-acetone to give the above-titled compound as pale yellow powder (0.63 g).
IR(KBr)$cm^{-1}$: 3440, 2950, 2852, 1485, 1466, 1380, 1116
NMR(90MHz, $CDCl_3$) δ: 0.87 (3H, t), 1.1 to 1.2 (42H, m), 3.20 to 3.57 (8H, m), 4.92 (2H, br-t), 7.94 (1H, t), 8.97 (2H, d), 9.88 (1H, s)

Effects of the Invention

The effects of the compounds of the present invention are described in detail by the following Test Examples.

Test Example 1

The effect of the compound of this invention in inhibiting multiplication of myelogenous leukemia cells (HL-60) is shown in Table 1. The assay was conducted as follows.

HL-60 cells (myelogenous leukemia cell lines) were suspended ($2 \times 10^5$cells/mℓ) in a GIT culture medium (Wako Pure Chemical Industries, Ltd.). Each hole of a 96-hole Microwell plate was inoculated with 0.2 mℓ of the suspension. The plate was incubated statically for 20 hours (37° C and 5% $CO_2$), to which was added 1 μCi of [$^3$H]thymidine(5 Ci/mmol.), and the incubation was continued for further 4 hours. For determination of the amount of thymidine incorporated into cells, acid-insoluble fractions were collected with a glass-filter, and the radioactivity was determined by means of a liquid scintillation counter. The concentration of the drug required for reducing the radioactivity incorporated into cells to 50% of the control group was assumed to be $IC_{50}$ value of the compound.

Table 1

| Test Compound | Inhibition of Propagation of HL-60 cells($IC_{50}$ : μg/mℓ) |
|---|---|
| Compound of Working Example 1 | 1.25 |
| Compound of Working Example 2 | 0.62 |
| Compound of Working Example 3 | 1.25 |
| Compound of Working Example 4 | 1.25 |
| Compound of Working Example 5 | 5 |
| Compound of Working Example 7 | 2.5 |
| Compound of Working Example 8 | 1.25 |

Test Example 2

Antitumor action of the compound of Working Examples

ICR mice (a group consisting of 5 mice) were inoculated intraperitoneally with 1 x $10^5$ Sarcoma 180 cells per mouse. Then, 0.33 mg/mouse of a compound dissolved in physiological saline was intraperitoneally administered three times, i.e. 1 hour, 1 day and 2 days after the inoculation. Shown in Table 2 are the lifespan prolongation ratio against the control group without treatment and the number of surviving mice on the 60th day after the initiation of the test.

Table 2

| Test Compound | Life-span prolongation ratio (T/C,%) *1 | 60th day number of survivors/number of mice tested |
|---|---|---|
| Compound of Working Example 1 | 295 | 2/5 |
| Compound of Working Example 3 | 342 | 2/5 |
| Control group | 100 | 0/5 |

*1 Calculation was made only on died mice

Test Example 3

C3H/He mice (a group consisting of 5 mice) were inoculated intraperitoneally with 1 x $10^4$ MM46 cells per mouse, and given 0.25 mg/mouse of a drug for 4 consecutive days starting from the second day after the inoculation. Shown in Table 3 are the life-span prolongation ratio against the control group without treatment (provided that calculation was made on mice whose survival days were not longer than 60 days) and the number of surviving mice on the 60th day after the inoculation of MM46 cells.

Table 3

| Test Compound | Life-span prolongation ratio (T/C,%) | 60th day No. of survivors/ No. of mice tested |
|---|---|---|
| Compound of Working Example 1 | - | 5/5 |
| Control group | 100 | 0/5 |

Test Example 4

Antitumor action of the compound of Working Example

ICR mice (a group consisting of 6 mice) were inoculated with 1 x $10^5$ Sarcoma 180 cells per mouse in the dorsal subcutis, and then 0.3 mg/mouse of a test compound dissolved in physiological saline was orally administered 9 times starting from the 8th day (8th to 10th, 13th to 17th and 20th days). On the 21st day, a tumor was enucleated, and the weight of the tumor was measured. The ratio of tumor weight of the treated group against that of the control group without treatment by drug is shown in Table 4.

Table 4

| Test Compound | Weight of tumor (T/C,%) |
|---|---|
| Compound of Working Example 3 | 24 |
| Control group | 100 |

As stated in the foregoing, the present invention provides drugs effective as antitumor agents.

## Claims

1. A compound of the formula:
$R^1$-$OCH_2CH_2CH_2O$-$(CH_2)_n$-$R^2$ (I)
wherein $R^1$ stands for a higher alkyl group; $R^2$ stands for a primary to tertiary amino group or a quaternary ammonium group; and n denotes an integer of 3 to 10; or a salt thereof.

2. A compound according to claim 1, wherein $R^1$ stands for an alkyl group having 8 to 22 carbon atoms which is unsubstituted or substituted by $C_{3-8}$ cycloalkyl, phenyl, halogen, cyano, ethynyl, oxo or $C_{1-4}$ alkoxy, said phenyl group being unsubstituted or substituted by $C_{1-4}$ alkyl, $C_{1-4}$ alkoxy, hydroxy, nitro or halogen;
$R^2$ stands for (i) a group represented by the formula:

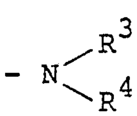

wherein $R^3$ and $R^4$ independently stand for hydrogen or $C_{1-5}$ alkyl, or both taken together with the adjacent nitrogen atom form a cyclic amino group selected from the class consisting of pyrrolidino, piperidino, piperazino and morpholino, said cyclic amino group being unsubstituted or substituted by $C_{1-4}$ alkyl, hydroxy, hydroxyethyl, aminoethyl, carboxy, $C_{1-4}$ alkoxycarbonyl, carbamoyl or ureido, or (ii) a quaternary ammonium group represented by the formula:

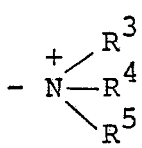

wherein $R^3$, $R^4$ and $R^5$ independently stand for hydrogen or $C_{1-5}$ alkyl, or $R^3$, $R^4$ and $R^5$ taken together with the adjacent nitrogen atom form a cyclic ammonium group selected from the class consisting of pyridinio, oxazolio, thiazolio, pyridazinio, quinolinio, isoquinolinio, 1-[$C_{1-4}$ alkyl]pyrrolidinio, 1-[$C_{1-4}$ alkyl]piperidinio, N-[$C_{1-4}$ alkyl]morpholinio and 1-[$C_{1-4}$ alkyl]piperazinio, said cyclic ammonium group being unsubstituted or substituted by $C_{1-4}$ alkyl, hydroxy, hydroxyethyl, aminoethyl, carboxy, $C_{1-4}$ alkoxycarbonyl, carbamoyl or ureido;
n denotes an integer of 3 to 10;
or a pharmaceutically acceptable salt thereof, with proviso that said compound represented by the formula (I) forms a pharmaceutically acceptable salt with an anion or an intramolecular salt with an anion in the molecule of said compound when $R^2$ stands for a quaternary ammonium group.

3. A compound according to claim 1, wherein $R^1$ stands for $C_{8-22}$ alkyl.

4. A compound according to claim 1, wherein $R^1$ stands for n-octadecyl.

5. A compound according to claim 1, wherein $R^2$ stands for a quaternary ammonium group.

6. A compound according to claim 1, wherein $R^2$ stands for trimethylammonium group.

7. A compound according to claim 1, wherein n denotes an integer of 3 to 8.

8. A compound according to claim 1, wherein n denotes an integer of 4 to 6.

9. A compound according to claim 1, which is an acid anion salt of N-[4-[3-(octadecyloxy)propoxy]butyl-N,N,N-trimethylammonium.

10. A compound according to claim 1, which is an acid anion salt of 6-[3-(octadecyloxy)propoxy]hexyl-N,N,N-trimethylammonium.

11. A compound according to claim 1, which is 1-N-[6-[3-(octadecyloxy)propoxy]hexyl]pyridinio-3-carboxylate.

12. A compound according to claim 1, which is an acid anion salt of N-[8-[3-(octadecyloxy)propoxy]octyl]thiazolium.

13. A pharmaceutical composition which comprises, as an active ingredient, an effective amount of a compound as claimed in any one of claims 1 to 12, or a salt thereof, in association with a pharmaceutically acceptable carrier or excipient therefor.

14. A compound as claimed in any one of claims 1 to 12 or a pharmaceutical composition as claimed in claim 13 for use in therapeutical treatment of a mammal.

15. A process for producing a compound of the formula:

$R^1\text{-}OCH_2CH_2CH_2O\text{-}(CH_2)_n\text{-}R^2$ (I)

wherein $R^1$ stands for a higher alkyl group; $R^2$ stands for a primary to tertiary amino group or a quaternary ammonium group; and n denotes an integer of 3 to 10; or a salt thereof, which comprises reacting a compound of the formula:

$R^1\text{-}OCH_2CH_2CH_2O\text{-}(CH_2)_n\text{-}Y$

wherein Y stands for benzenesulfonyloxy, lower alkylbenzenesulfonyloxy, lower alkanesulfonyloxy or halogen; and the other symbols are of the same meaning as defined above; with an amine, and if desired, converting the thus obtained compound of the formula (I) into a salt thereof.